# EUROPEAN PATENT APPLICATION

(11) **EP 4 624 560 A1**
(43) Date of publication of application: **01.10.2025**
(21) Application number: 25162330.2
(22) Date of filing: 07.03.2025
(51) Int. Cl.: C12M 1/00, C12M 1/24, C12M 1/26, B01L 3/00, C12Q 1/04

(54) **CULTURE VESSEL, CULTURE KIT, AND METHOD FOR PERFORMING FALLING BACTERIA TEST OF MICROORGANISMS**

(30) Priority: 19.03.2024 JP 2024043848
(71) Applicant: Yokogawa Electric Corporation, Tokyo 180-8750 (JP)
(72) Inventor: Mitsumori, Yuuji, Musashino-shi, Tokyo 180-8750 (JP); Taguchi, Tomoyuki, Musashino-shi, Tokyo 180-8750 (JP)
(74) Representative: Grünecker Patent- und Rechtsanwälte PartG mbB

(57) **Abstract**

Provided is a culture vessel, a culture kit, and a method for performing a falling bacteria test of microorganisms capable of eliminating a complexity of an operation of the test. A culture vessel of the present disclosure is used for a falling bacteria test of microorganisms. The culture vessel includes a vessel main body portion configured to internally house a culture fluid. The vessel main body portion has a top portion, a body, and a bottom portion. The top portion is provided with a first opening portion configured to receive the microorganisms falling from an environment above the culture vessel in an inside of the vessel main body portion. The top portion or the body is provided with a second opening portion configured to pour the culture fluid. The culture vessel further includes a first lid that closes the first opening portion and a second lid that closes the second opening portion.

## Description

### BACKGROUND

### Description of Related Art

The present disclosure relates to a culture vessel and a culture kit used for a falling bacteria test of microorganisms, and a method for performing the falling bacteria test of microorganisms using the culture vessel.

### Background Art

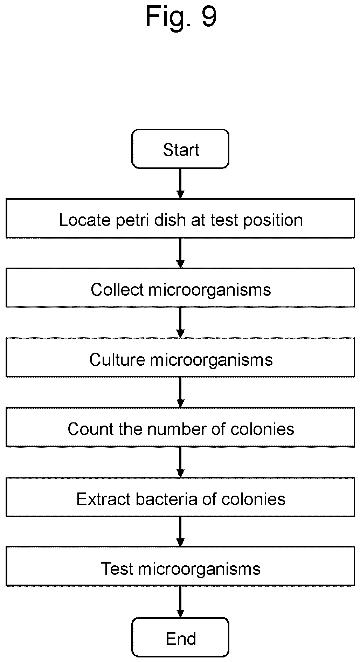

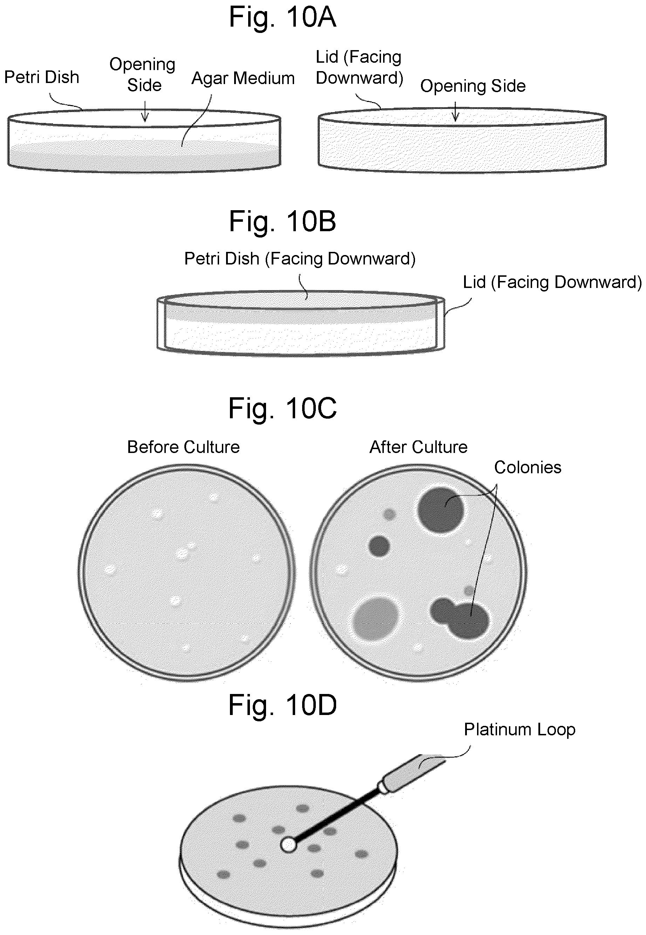

Conventionally, there has been known a falling bacteria method as a testing method of microorganisms in an environment. The falling bacteria method is also referred to as Koch method, and is a method in which an agar medium (plate medium) of a certain size is opened for a certain period of time, then microorganisms fallen on a surface of the agar medium is cultured, and a test of the microorganisms is performed using colonies of the grown microorganisms. In this method, the number of the grown colonies is measured as the number of bacteria per unit time, and bacteria are extracted from the colonies to perform tests, such as a confirmation of characteristics of the cultured microorganisms. FIG. 9 is a flowchart schematically illustrating a process of a falling bacteria test using the conventional falling bacteria method, and FIGS. 10A to 10D are schematic process drawings illustrating the falling bacteria test using the conventional falling bacteria method.

In the falling bacteria test using the conventional falling bacteria method, as illustrated in the flowchart of FIG. 9, first, a petri dish in which an agar medium is put is located at a test position in a state where a lid is opened (locate petri dish at test position) as illustrated in FIG. 10A. Next, until a certain period of time elapses after locating the petri dish at the test position, the petri dish is maintained to be located at the test position in the state where the lid is opened. Thus, microorganisms in an environment above the petri dish fall on the agar medium in the petri dish. Then, as illustrated in FIGS. 10A and 10B, the petri dish facing downward is put into the lid facing downward so as to have the agar medium in the upper side, thereby closing the lid of the petri dish. Thus, the microorganisms are collected (collect microorganisms). Next, the petri dish with the closed lid is placed in an incubator (not illustrated) set to a temperature range necessary for culturing microorganisms to be cultured, and maintained for a predetermined period of time, thereby culturing the microorganisms in solid phase on the agar medium in the petri dish as illustrated in FIG. 10C (culture microorganisms). Next, the number of colonies of the cultured microorganisms is counted (count the number of colonies). Next, as illustrated in FIG. 10D, bacteria are extracted from the colonies using a platinum loop (extract bacteria of colonies). Next, a test, such as a characteristic confirmation of the cultured microorganisms, is performed using the colonies from which bacteria have been extracted (test microorganisms).

As a conventional technique relating to the culture and the test of microorganisms, such as a falling bacteria test as described above, for example, there has been known a technique relating to a film-type medium for culturing microorganisms (JP 2007-20434 A). This technique can provide a medium that eliminates a labor of medium preparation and is good in preservation, saved in space, and saved in disposal amount.

### SUMMARY

In the conventional falling bacteria test, as described above, to perform the test, such as a characteristic confirmation of the cultured microorganisms, the bacteria extraction from the colony is necessary. Since the operation of bacteria extraction is complicated, it takes time and effort.

The present disclosure is made in consideration of such a point, and provides a culture vessel and a culture kit used for a falling bacteria test of microorganisms and a method for performing the falling bacteria test of microorganisms, which are capable of eliminating a complexity of the operation of the test.

To solve the above-described problem, a culture vessel of the present disclosure is a culture vessel used for a falling bacteria test of microorganisms. The culture vessel comprises a vessel main body portion configured to internally house a culture fluid. The vessel main body portion has a top portion, a body, and a bottom portion. The top portion is provided with a first opening portion configured to receive the microorganisms falling from an environment above the culture vessel in an inside of the vessel main body portion. At least one of the top portion or the body is provided with a second opening portion configured to pour the culture fluid. The culture vessel further includes a first lid that closes the first opening portion and a second lid that closes the second opening portion.

A culture kit of the present disclosure is a culture kit used for a falling bacteria test of microorganisms. The culture kit comprises the culture vessel described above, a microorganism capturing filter, and an attachment material used for attaching the microorganism capturing filter to an area in which the microorganisms fall via the first opening portion on a bottom surface inside the vessel main body portion of the culture vessel.

A method for performing a falling bacteria test of microorganisms of the present disclosure is a method for performing a falling bacteria test of microorganisms using the culture vessel described above. The method comprises: locating the culture vessel at a test position with the first opening portion opened; collecting microorganisms in an environment above the culture vessel falling in an inside of the vessel main body portion via the first opening portion until a certain period of time elapses after locating the culture vessel at the test position; supplying a liquid medium to the inside of the vessel main body portion via at least one of the first opening portion or the second opening portion after collecting the microorganisms; culturing the microorganisms in the liquid medium supplied to the inside of the vessel main body portion to obtain a culture fluid; extracting the culture fluid to an outside of the culture vessel from the inside of the vessel main body portion via the second opening portion; and testing the microorganisms using the culture fluid extracted to the outside.

### EFFECT

The present disclosure can eliminate the complexity of the operation of the test.

### BRIEF DESCRIPTION OF THE DRAWINGS

FIG. 1A is a schematic perspective view illustrating a state of collecting microorganisms in a culture vessel according to a first embodiment, and FIG. 1B is a schematic perspective view illustrating a state of extracting a culture fluid to an outside according to the first embodiment;
FIG. 2 is a flowchart schematically illustrating a process of a method for performing a falling bacteria test of microorganisms according to the first embodiment;
FIG. 3A is a schematic perspective view illustrating a state of collecting microorganisms in a culture vessel according to a second embodiment, and FIG. 3B is a schematic perspective view illustrating a state of extracting a culture fluid to an outside according to the second embodiment;
FIG. 4A is a schematic perspective view illustrating an initial state of a culture kit according to a third embodiment, and FIG. 4B is a schematic perspective view illustrating a state of extracting a culture fluid to an outside according to the third embodiment;
FIG. 5 is a schematic perspective view illustrating a culture vessel according to a modification of the first embodiment;
FIG. 6 is a schematic perspective view illustrating a culture vessel according to a modification of the second embodiment;
FIG. 7 is a schematic perspective view illustrating a culture kit according to a modification of the third embodiment;
FIG. 8A and FIG. 8B are schematic perspective views illustrating a second lid of the culture vessel according to the modification of the first embodiment;
FIG. 9 is a flowchart schematically illustrating a process of a falling bacteria test using a conventional falling bacteria method; and
FIG. 10A to FIG. 10D are schematic process drawings illustrating the falling bacteria test using the conventional falling bacteria method.

### DETAILED DESCRIPTION OF THE EMBODIMENTS

The following describes embodiments according to a culture vessel, a culture kit, and a method for performing a falling bacteria test of microorganisms of the present disclosure.

### [First Embodiment]

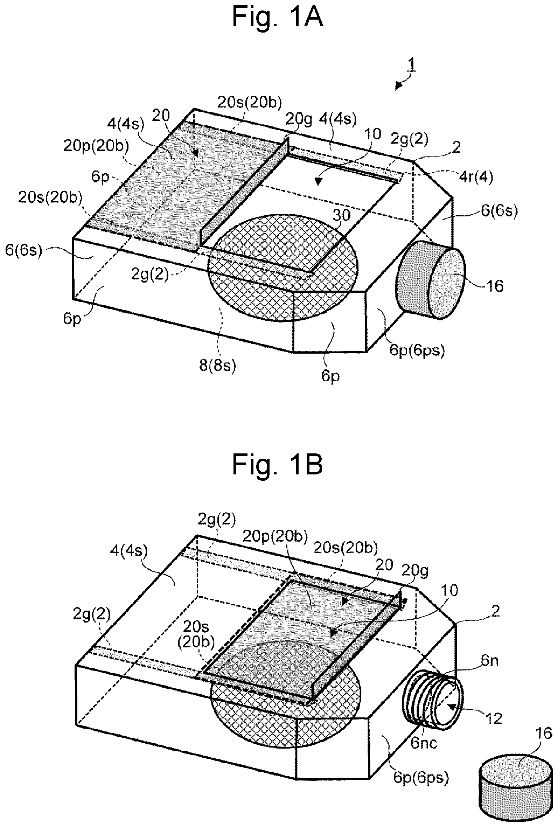

First, a culture vessel and a method for performing a falling bacteria test of microorganisms according to the first embodiment are described. FIG. 1A is a schematic perspective view illustrating a state of collecting microorganisms in a culture vessel according to the first embodiment, and FIG. 1B is a schematic perspective view illustrating a state of extracting a culture fluid to an outside according to the first embodiment.

As illustrated in FIG. 1A and FIG. 1B, a culture vessel 1 according to the first embodiment is a culture vessel 1 used for the falling bacteria test of microorganisms, and includes a vessel main body portion 2 that can internally house a culture fluid. The vessel main body portion 2 has a top portion (upper portion) 4, a body 6, and a bottom portion 8. A schematic shape of the vessel main body portion 2 is a flat prism having a surface 4s of the top portion 4 as a top surface, a surface 8s of the bottom portion 8 as a bottom surface, and a surface 6s of the body 6 as a side surface. The top portion 4 and the bottom portion 8 have the same schematic shape in a plate shape in which corners in a side of one short side of a rectangular are cut off, and the body 6 has a schematic shape in which a plurality of rectangular plate-shaped members 6p coupling a plurality of sides of the top portion 4 to respective sides of the bottom portion 8 corresponding thereto are joined in a circumferential direction. The top portion 4 is provided with a first opening portion 10 configured to receive microorganisms falling from an environment above the culture vessel 1 in an inside of the vessel main body portion 2. The first opening portion 10 has a rectangular shape in which short sides and long sides are parallel to long sides and short sides of the top portion 4, respectively. Among the plurality of plate-shaped members 6p of the body 6, a plate-shaped member 6p coupling the short sides of the top portion 4 and the bottom portion 8 in the side in which the corners are cut off is provided with a neck portion 6n projecting outward from a surface 6ps of the plate-shaped member 6p. The neck portion 6n of the body 6 is provided with an openable/closable second opening portion 12 configured to pour the culture fluid.

The culture vessel 1 further includes a slide lid 20 that slides and moves in a direction along the short side of the first opening portion 10 to open and close the first opening portion 10 as a first lid that opens and closes the first opening portion 10. The slide lid 20 has a lid body 20b and a grip portion 20g. The lid body 20b includes a rectangular plate-shaped lid portion 20p and two sliding portions 20s extending from two short sides of the lid portion 20p to respective opposite sides of the lid portion 20p. The short sides and long sides of the lid portion 20p are parallel to the short sides and the long sides of the first opening portion 10, respectively. So as to allow the lid portion 20p to completely cover and close the first opening portion 10, the short side of the lid portion 20p has a dimension slightly longer than the short side of the first opening portion 10, and the long side of the lid portion 20p has a dimension same as the long side of the first opening portion 10. The sliding portion 20s has a rectangular plate shape in which two short sides extend from ends of the respective two long sides of the lid portion 20p in a direction parallel to the long sides, and a long side in the lid portion 20p side of the sliding portion 20s is the same as the short side of the lid portion 20p. The grip portion 20g projects upward from a surface of the lid portion 20p at the long side in the second opening portion 12 side of the lid portion 20p.

The top portion 4 of the vessel main body portion 2 has a back surface 4r provided with two guiding portions 2g that guide the respective two sliding portions 20s of the slide lid 20 so as to allow the slide lid 20 to slide and move to the second opening portion 12 side and an opposite side thereof in a direction along the short side of the first opening portion 10. The respective two guiding portions 2g extend from end positions in the second opening portion 12 side of the two short sides of the first opening portion 10 of the top portion 4 to positions of the short sides in the opposite side of the second opening portion 12 of the top portion 4 in a direction parallel to the short sides. The guiding portion 2g has a rectangular plate shape in which short sides are parallel to the long sides of the first opening portion 10 and long sides are parallel to the short sides of the first opening portion 10. The guiding portion 2g is disposed such that in plan view of the top portion 4, the whole short side of the first opening portion 10 overlaps with the long side in the first opening portion 10 side of the guiding portion 2g. The long side portion in the opposite side of the first opening portion 10 of the guiding portion 2g is secured to the back surface 4r of the top portion 4. In the slide lid 20, the lid body 20b is disposed inside the vessel main body portion 2 such that the respective two sliding portions 20s are interposed between the back surface 4r of the top portion 4 and the two guiding portions 2g, and the grip portion 20g projects to the outside of the vessel main body portion 2 via the first opening portion 10.

The culture vessel 1 further includes a cap 16 (second lid) that can be attached to and removed from the second opening portion 12 such that the second opening portion 12 can be opened and closed. The cap 16 is provided with a thread (not illustrated) on an inner portion, the neck portion 6n at which the second opening portion 12 is provided is also provided with a thread 6nc on an outer portion, and these threads mesh with one another. By putting the cap 16 over the neck portion 6n, meshing the thread of the cap 16 with the thread 6nc of the neck portion 6n, and tightening the cap 16, the cap 16 can be attached to the second opening portion 12 and the second opening portion 12 can be closed. By reversely winding and loosening the cap 16 from the state, the cap 16 can be removed from the second opening portion 12 and the second opening portion 12 can be opened.

The culture vessel 1 further includes a microorganism capturing filter 30 attached to an area in which microorganisms fall via the first opening portion 10 on the bottom surface inside the vessel main body portion 2. The microorganism capturing filter 30 is a membrane filter having a filter hole size of 0.45 µm or less, consists of a material without a negative effect on microorganisms, and has a structure without a negative effect on microorganisms.

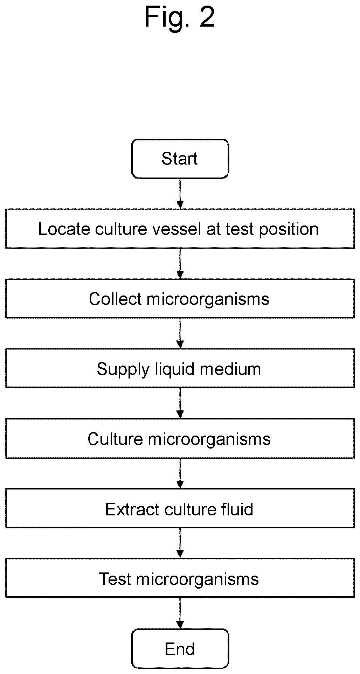

In a method for performing a falling bacteria test of microorganisms according to the first embodiment, the falling bacteria test of microorganisms is performed using the culture vessel 1 according to the first embodiment. FIG. 2 is a flowchart schematically illustrating a process of the method for performing the falling bacteria test of microorganisms according to the first embodiment.

In the method according to the first embodiment, as illustrated in the flowchart of FIG. 2, first, the cap 16 is attached to the second opening portion 12 of the culture vessel 1 to close the second opening portion 12, then the slide lid 20 is slid and moved to the opposite side of the second opening portion 12 to open the first opening portion 10, and the culture vessel 1 is located at a test position as illustrated in FIG. 1A (locate culture vessel at test position). Next, until a certain period of time elapses after the culture vessel 1 is located at the test position, the culture vessel 1 is maintained to be located at the test position. Thus, microorganisms in an environment above the culture vessel 1 fall on the microorganism capturing filter 30 inside the vessel main body portion 2 via the first opening portion 10, thereby capturing the microorganisms by the microorganism capturing filter 30. Then, the slide lid 20 is slid and moved to the second opening portion 12 side to close the first opening portion 10 once. Thus, the microorganisms are collected inside the vessel main body portion 2 (collect microorganisms).

Next, the slide lid 20 is slid and moved to the opposite side of the second opening portion 12 to open the first opening portion 10 again, and a liquid medium (not illustrated) is supplied to the inside of the vessel main body portion 2 via the first opening portion 10 (supply liquid medium). At this time, to immerse the whole microorganism capturing filter 30 in the liquid medium, for example, the liquid medium is supplied to the inside of the vessel main body portion 2 in an amount of approximately a quarter of a total volume of the inside of the vessel main body portion 2. By supplying the liquid medium, the microorganisms captured by the microorganism capturing filter 30 are separated from the microorganism capturing filter 30, and float in the liquid medium.

Next, the slide lid 20 is slid and moved to the second opening portion 12 side to close the first opening portion 10, the culture vessel 1 is placed in an incubator (not illustrated) set to a temperature range necessary for culturing microorganisms to be cultured, and maintained for a predetermined period of time in a state of being placed in the incubator, thereby culturing the microorganisms in the liquid medium supplied to the inside of the vessel main body portion 2 and obtaining a culture fluid (culture microorganisms).

Next, as illustrated in FIG. 1B, the cap 16 is removed from the second opening portion 12 of the culture vessel 1 to open the second opening portion 12, and the culture fluid is extracted from the inside of the vessel main body portion 2 to an outside of the culture vessel 1 via the second opening portion 12 (extract culture fluid). At this time, for example, the culture fluid is poured into a test vessel (not illustrated), such as a centrifuge tube, from the inside of the vessel main body portion 2 via the second opening portion 12, and the culture fluid is directly extracted to the test vessel.

Next, the microorganisms are tested using the culture fluid extracted outside (test microorganisms). At this time, as the test of the microorganisms, a characteristic confirmation, a genetic test, or the like of the microorganisms is performed. Examples of a method of the genetic test of the microorganisms include a method in which nucleic acids are extracted from the cultured microorganisms included in the culture fluid extracted to the test vessel, such as a centrifuge tube, then the nucleic acids are amplified using a PCR method or the like, and genes of the amplified nucleic acids are analyzed to identify the microorganisms. As described above, the falling bacteria test of microorganisms is performed using the culture vessel 1 according to the first embodiment.

The culture vessel 1 according to the first embodiment is provided with the first opening portion 10 in the top portion 4 of the vessel main body portion 2, and the first opening portion 10 is configured to receive microorganisms falling from an environment in the inside of the vessel main body portion 2. The body 6 of the vessel main body portion 2 is provided with the openable/closable second opening portion 12 configured to pour the culture fluid. Furthermore, the culture vessel 1 according to the first embodiment includes the slide lid 20 (first lid) that opens and closes the first opening portion 10 and the cap 16 (second lid) attachable to and removable from the second opening portion 12 to allow the second opening portion 12 to be opened and closed. Accordingly, in the method for performing the falling bacteria test of microorganisms according to the first embodiment, by the use of the culture vessel 1 according to the first embodiment, the first opening portion 10 is opened and microorganisms in an environment fall in an inside of the vessel main body portion 2 via the first opening portion 10, thereby allowing collecting the microorganisms by the falling bacteria method. After collecting the microorganisms, the liquid medium is supplied to the inside of the vessel main body portion 2 via the first opening portion 10, and then the microorganisms are cultured in the liquid medium supplied to the inside of the vessel main body portion 2, thereby allowing obtaining the culture fluid. Furthermore, the culture fluid can be extracted to an outside of the culture vessel 1 from the inside of the vessel main body portion 2 via the second opening portion 12, and the test, such as a characteristic confirmation and a genetic test of the microorganisms, can be performed using the culture fluid extracted to the outside. Therefore, different from the conventional falling bacteria test, it is not necessary to extract bacteria from the colonies of the cultured microorganisms for performing the test, such as a characteristic confirmation and a genetic test of the microorganisms. Since the culture fluid can be poured into a test vessel, such as a centrifuge tube, from the inside of the vessel main body portion 2 via the second opening portion 12 and the culture fluid can be directly extracted to the test vessel, it is not necessary to extract the culture fluid from the inside of the vessel main body portion 2 to the test vessel using a pipetman or the like. Accordingly, a complexity of the operation of the test can be eliminated. A contamination risk in the process of microbial treatment for the test can be reduced. Furthermore, those other than highly specialized workers can perform the test. Additionally, since not the solid-phase culture but the liquid culture is performed in the culture of the microorganisms, the time taken for the culture to grow the microorganisms can be reduced, thus allowing reducing the test time.

Furthermore, the culture vessel 1 according to the first embodiment further includes the microorganism capturing filter 30 attached to the area in which microorganisms fall via the first opening portion 10 on the bottom surface inside the vessel main body portion 2. The microorganism capturing filter 30 is a membrane filter having a filter hole size of 0.45 µm or less, consists of a material without a negative effect on microorganisms, and has a structure without a negative effect on microorganisms. Therefore, in the method for performing the falling bacteria test of microorganisms according to the first embodiment, when microorganisms falling in the inside of the vessel main body portion 2 via the first opening portion 10 are collected, the microorganisms falling on the microorganism capturing filter 30 can be captured by the microorganism capturing filter 30, thereby allowing suppressing the collected microorganisms to be damaged. Accordingly, the microorganisms can be reliably cultured. Since the microorganism capturing filter 30 is attached to the area in which microorganisms fall, the position of the microorganism capturing filter 30 is not displaced, and the microorganisms reliably fall on the microorganism capturing filter 30 and can be captured.

### [Second Embodiment]

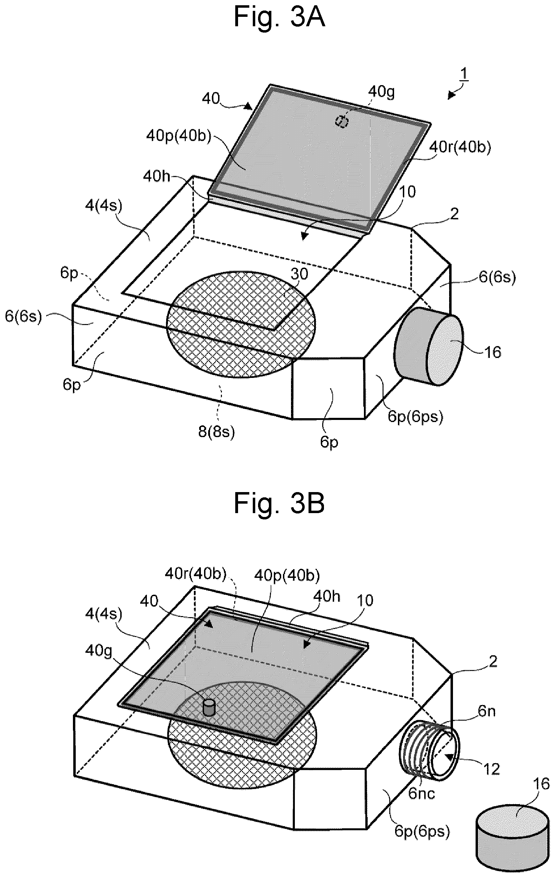

Next, a culture vessel and a method for performing a falling bacteria test of microorganisms according to the second embodiment are described mainly for the difference from the first embodiment. FIG. 3A is a schematic perspective view illustrating a state of collecting microorganisms in a culture vessel according to the second embodiment, and FIG. 3B is a schematic perspective view illustrating a state of extracting a culture fluid to an outside according to the second embodiment.

As illustrated in FIG. 3A and FIG. 3B, a culture vessel 1 according to the second embodiment includes a vessel main body portion 2 similar to that of the culture vessel according to the first embodiment excluding that a first opening portion 10 is provided at a position close to the center of a top portion 4 and guiding portions 2g are not provided. The culture vessel 1 according to the second embodiment further includes a swing lid 40 as a first lid that opens and closes the first opening portion 10 different from the culture vessel according to the first embodiment. The swing lid 40 opens and closes the first opening portion 10 by rotating a lid body 40b to an opposite side of the top portion 4 and the top portion 4 side with a hinge 40h as an axis like a swing door. The swing lid 40 includes the rectangular plate-shaped lid body 40b, the hinge 40h that couples a portion of one side of the lid body 40b to a portion of one side of the first opening portion 10 of the top portion 4, and a grip portion 40g. The lid body 40b includes a rectangular plate-shaped lid portion 40p and a rib 40r that is provided on a surface in the first opening portion 10 side of the lid portion 40p and engaged with a peripheral portion of the first opening portion 10 in the top portion 4. The culture vessel 1 according to the second embodiment further includes a cap 16 (second lid) similar to that of the culture vessel according to the first embodiment. The culture vessel 1 according to the second embodiment, similarly to the culture vessel according to the first embodiment, further includes a microorganism capturing filter 30 attached to an area in which microorganisms fall via the first opening portion 10 on the bottom surface inside the vessel main body portion 2.

In a method for performing a falling bacteria test of microorganisms according to the second embodiment, the falling bacteria test of microorganisms is performed using the culture vessel 1 according to the second embodiment. In this case, when the first opening portion 10 is opened, the lid body 40b of the swing lid 40 is rotated to the opposite side of the top portion 4 around the hinge 40h as the axis. When the first opening portion 10 is closed, the lid body 40b of the swing lid 40 is rotated to the top portion 4 side around the hinge 40h as the axis, and the rib 40r of the lid body 40b is engaged with the peripheral portion of the first opening portion 10 in the top portion 4. Except these points, the falling bacteria test of microorganisms is performed similarly to the method for performing the falling bacteria test of microorganisms according to the first embodiment. Accordingly, the culture vessel and the method for performing the falling bacteria test of microorganisms according to the second embodiment provide the operational advantages similar to those of the first embodiment.

### [Third Embodiment]

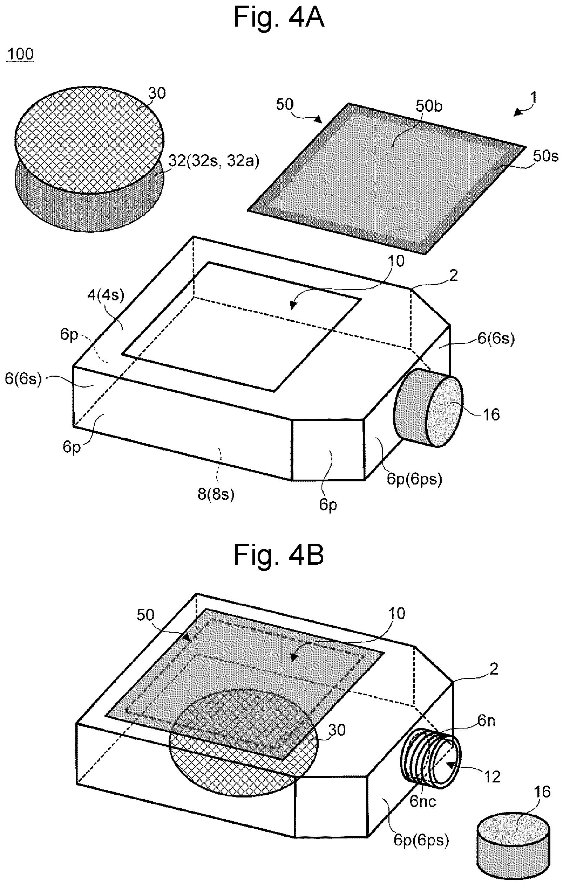

Next, a culture kit and a method for performing a falling bacteria test of microorganisms according to the third embodiment are described mainly for the difference from the first embodiment. FIG. 4A is a schematic perspective view illustrating a state of collecting microorganisms in the culture kit according to the third embodiment, and FIG. 4B is a schematic perspective view illustrating a state of extracting a culture fluid to an outside according to the third embodiment.

As illustrated in FIG. 4A and FIG. 4B, a culture kit 100 according to the third embodiment includes a culture vessel 1, a microorganism capturing filter 30, and an attachment material 32 used for attaching the microorganism capturing filter 30 to an area in which microorganisms fall via a first opening portion 10 on a bottom surface inside a vessel main body portion 2 of the culture vessel 1. The culture vessel 1 includes a vessel main body portion 2 similar to that of the culture vessel according to the first embodiment excluding that the first opening portion 10 is provided at a position close to the center of a top portion 4 and guiding portions 2g are not provided. The culture vessel 1 are different from the culture vessel according to the first embodiment in that the culture vessel 1 further includes a sheet lid 50 as a first lid that opens and closes the first opening portion 10. The sheet lid 50 is attached to a surface 4s of the top portion 4 so as to cover the first opening portion 10, and peeled off from there, thereby opening and closing the first opening portion 10. The sheet lid 50 includes a rectangular lid body sheet 50b having a size enough to cover the first opening portion 10, and an adhesive seal 50s provided on an outer peripheral portion of an attachment surface of the lid body sheet 50b. The culture vessel 1 further includes a cap 16 (second lid) similar to that of the culture vessel according to the first embodiment. The microorganism capturing filter 30 is a microorganism capturing filter 30 similar to that of the culture vessel according to the first embodiment. The attachment material 32 is a double-sided adhesive sheet including a substrate sheet 32s and two adhesive layers 32a provided on respective both surfaces of the substrate sheet.

In a method for performing a falling bacteria test of microorganisms according to the third embodiment, the falling bacteria test of microorganisms is performed using the culture kit 100 according to the third embodiment. In this case, before the culture vessel 1 is located at a test position, the microorganism capturing filter 30 is attached at the area in which microorganisms fall via the first opening portion 10 on the bottom surface inside the vessel main body portion 2 of the culture vessel 1 using the attachment material (double-sided adhesive sheet) 32. When the first opening portion 10 is closed, the adhesive seal 50s of the sheet lid 50 is adhered to the peripheral portion around the first opening portion 10 in the surface 4s of the top portion 4, thereby attaching the sheet lid 50 to the surface 4s of the top portion 4 so as to cover the first opening portion 10. Furthermore, when the first opening portion 10 is closed once and then the first opening portion 10 is opened again, the adhesive seal 50s of the sheet lid 50 is peeled off from the surface 4s of the top portion 4, thereby removing the sheet lid 50 from the surface 4s of the top portion 4. Except these points, the falling bacteria test of microorganisms is performed similarly to the method for performing the falling bacteria test of microorganisms according to the first embodiment. Accordingly, the culture kit and the method for performing the falling bacteria test of microorganisms according to the third embodiment provide the operational advantages similar to those of the first embodiment.

### [Modifications]

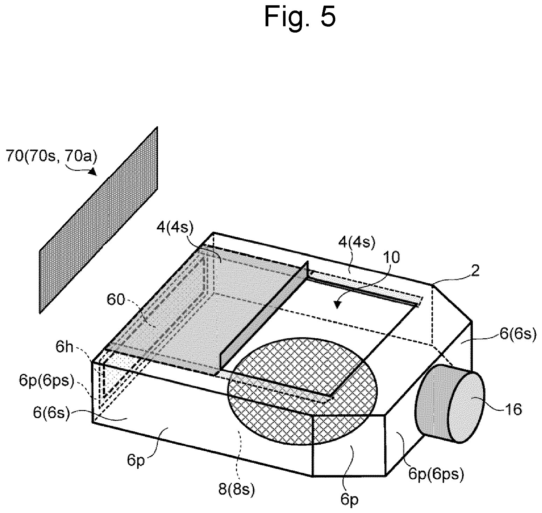

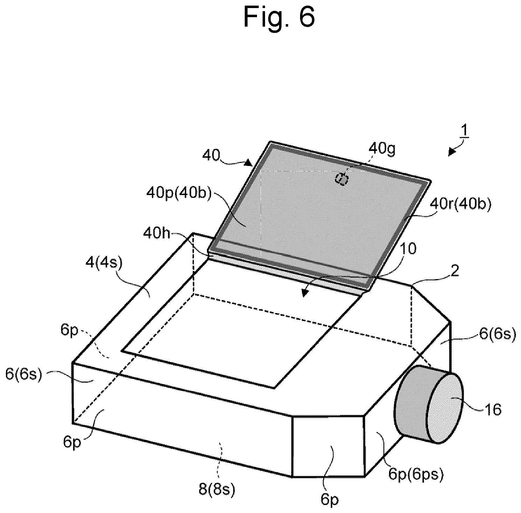

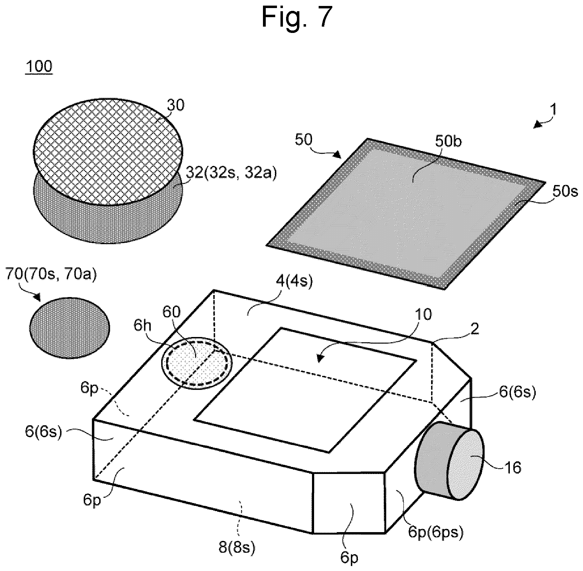

Next, modifications of the first to the third embodiments are described mainly for the differences from the first to the third embodiments, respectively. FIG. 5 is a schematic perspective view illustrating a culture vessel according to a modification of the first embodiment. FIG. 6 is a schematic perspective view illustrating a culture vessel according to a modification of the second embodiment. FIG. 7 is a schematic perspective view illustrating a culture kit according to a modification of the third embodiment.

As illustrated in FIG. 5, a culture vessel 1 according to the modification of the first embodiment is, different from the culture vessel according to the first embodiment, provided with a vent hole 6h in a plate-shaped member 6p in an opposite side of a cap 16 (second opening portion 12) in a body 6 of a vessel main body portion 2, and a hydrophobic filter 60 (air-permeable membrane material) is attached to a surface 6ps of the plate-shaped member 6p so as to cover the vent hole 6h. Furthermore, the culture vessel 1 further includes, different from the culture vessel according to the first embodiment, an air-impermeable cover 70 that opens and closes the vent hole 6h. The air-impermeable cover 70 is a one-sided adhesive sheet including a substrate sheet 70s and an adhesive layer 70a provided on one surface of the substrate sheet 70s. The adhesive layer 70a of the air-impermeable cover 70 is adhered to the surface 6ps of the plate-shaped member 6p to attach the air-impermeable cover 70 to the surface 6ps of the plate-shaped member 6p so as to cover the vent hole 6h and the hydrophobic filter 60, thereby allowing closing the vent hole 6h. Then, the adhesive layer 70a of the air-impermeable cover 70 is peeled off from the surface 6ps of the plate-shaped member 6p to remove the air-impermeable cover 70 from the surface 6ps of the plate-shaped member 6p, thereby allowing opening the vent hole 6h. Except these points, the culture vessel 1 is similar to the culture vessel according to the first embodiment.

A method for performing the falling bacteria test of microorganisms using the culture vessel 1 according to the modification of the first embodiment provides the effects similar to those of the first embodiment. Furthermore, in this method, by opening the vent hole 6h when the culture vessel 1 is located at the test position and microorganisms in an environment above the culture vessel 1 fall in an inside of the vessel main body portion 2 via the first opening portion 10, air flows from the inside of the vessel main body portion 2 to the outside via the vent hole 6h. Therefore, the microorganisms easily fall in an inside of the vessel main body portion 2 via the first opening portion 10. This allows reliably collecting the microorganisms in the environment. In a case where aerobic bacteria are collected as microorganisms, by opening the vent hole 6h when the microorganisms are cultured in the liquid medium supplied to the inside of the vessel main body portion 2, air can be taken into the liquid medium from the outside of the vessel main body portion 2 via the vent hole 6h without flowing out the liquid medium from the inside of the vessel main body portion 2 to the outside via the vent hole 6h. This allows promoting the culture of the aerobic bacteria. On the other hand, in a case where anaerobic bacteria are collected as microorganisms, by attaching the air-impermeable cover 70 to the surface 6ps of the plate-shaped member 6p to close the vent hole 6h when the microorganisms are cultured in the liquid medium supplied to the inside of the vessel main body portion 2, it can be avoided to take air into the liquid medium from the outside of the vessel main body portion 2 via the vent hole 6h, and thus it can be avoided that the culture of the anaerobic bacteria is inhibited.

As illustrated in FIG. 6, a culture vessel 1 according to the modification of the second embodiment is, different from the culture vessel according to the second embodiment, not provided with a microorganism capturing filter 30. Except this point, the culture vessel 1 is similar to the culture vessel according to the second embodiment. In a method for performing the falling bacteria test of microorganisms using the culture vessel 1 according to the modification of the second embodiment, different from the second embodiment, when the microorganisms falling in an inside of a vessel main body portion 2 via a first opening portion 10 are collected, instead of capturing the microorganisms falling on the microorganism capturing filter 30, the microorganisms falling on a bottom surface inside the vessel main body portion 2 are collected. Also in this case, similarly to the method for performing the falling bacteria test of microorganisms according to the second embodiment, after the microorganisms are collected, a liquid medium is supplied to the inside of the vessel main body portion 2 via the first opening portion 10, and then the microorganisms can be cultured in the liquid medium. Accordingly, the complexity of the operation of the test can be eliminated. The contamination risk can be reduced. Furthermore, those other than highly specialized workers can perform the test. Additionally, since not the solid-phase culture but the liquid culture is performed, the test time can be reduced.

As illustrated in FIG. 7, a culture kit 100 according to the modification of the third embodiment includes a culture vessel 1 different from the culture kit according to the third embodiment. The culture vessel 1 is provided with a first opening portion 10 at a position close to a cap 16 (second opening portion 12) side of a top portion 4. A vent hole 6h is provided at a position in an opposite side of the cap 16 (second opening portion 12) of the top portion 4 of the vessel main body portion 2, and a hydrophobic filter 60 (air-permeable membrane material) is attached to a surface 4s of the top portion 4 so as to cover the vent hole 6h. Furthermore, the culture vessel 1 further includes an air-impermeable cover 70 that opens and closes the vent hole 6h. The air-impermeable cover 70 is a one-sided adhesive sheet including a substrate sheet 70s and an adhesive layer 70a provided on one surface of the substrate sheet 70s. The adhesive layer 70a of the air-impermeable cover 70 is adhered to the surface 4s of the top portion 4 to attach the air-impermeable cover 70 to the surface 4s of the top portion 4 so as to cover the vent hole 6h and the hydrophobic filter 60, thereby allowing closing the vent hole 6h. Then, the adhesive layer 70a of the air-impermeable cover 70 is peeled off from the surface 4s of the top portion 4 to remove the air-impermeable cover 70 from the surface 4s of the top portion 4, thereby allowing opening the vent hole 6h. Except these points, the culture vessel 1 is similar to the culture vessel according to the third embodiment. A method for performing the falling bacteria test of microorganisms using the culture kit 100 according to the modification of the third embodiment provides, in addition to the effects similar to those of the third embodiment, the effects similar to those of the modification of the first embodiment.

Next, configurations of the culture vessel, the culture kit, and the method for performing the falling bacteria test of microorganisms according to the embodiments are described in more detail.

### 1. Culture Vessel

The culture vessel according to the embodiment is a culture vessel used for a falling bacteria test of microorganisms, and includes a vessel main body portion that can internally house a culture fluid. The vessel main body portion has a top portion, a body, and a bottom portion. The top portion is provided with a first opening portion configured to receive the microorganisms falling from an environment above the culture vessel in an inside of the vessel main body portion. The top portion or the body is provided with a second opening portion configured to pour the culture fluid. The culture vessel further includes a first lid that closes the first opening portion and a second lid that closes the second opening portion. That is, the culture vessel is a culture vessel used for both the liquid culture and the falling bacteria test of microorganisms.

While the material of the vessel main body portion is not specifically limited, the examples thereof include PET (polyethylene terephthalate). While the shape of the vessel main body portion is not specifically limited, the shape may be not bulky and facilitates carrying and locating at a test position in some embodiments, and specifically, for example, the vessel main body portion may have a shape having a bottom portion with a planar surface and a low body height (distance between the surface of the top portion and the surface of the bottom portion) in some embodiments.

While the first lid is not specifically limited insofar as the lid closes the first opening portion, for example, the first lid is a lid that opens and closes the first opening portion, and specifically, examples thereof include a slide lid that slides and moves to open and close the first opening portion like the slide lid according to first embodiment, a swing lid that rotates a lid body around a hinge as an axis to open and close the first opening portion like the swing lid according to the second embodiment, and a sheet lid that is attached to the surface of the top portion so as to cover the first opening portion and peeled off from there to open and close the first opening portion like the sheet lid according to the third embodiment. While the first lid may have a leak-proof structure to avoid leakage of the culture fluid from the inside of the vessel main body portion to the outside via the first opening portion, or does not need to have the leak-proof structure, the first lid may have the leak-proof structure in some embodiments. Examples of the leak-proof structure include a lid that includes a lid body with a rib engaged with the peripheral portion around the first opening portion in the top portion such that the leakage of the culture fluid can be avoided like the swing lid according to the second embodiment. Additionally, examples of the leak-proof structure include a lid that includes a rectangular lid body sheet having a size enough to cover the first opening portion and an adhesive seal provided on an outer peripheral portion of an attachment surface of the lid body sheet, in which the adhesive seal is attached to the peripheral portion around the first opening portion in the surface of the top portion such that the leakage of the culture fluid can be avoided like the sheet lid according to the third embodiment. While the slide lid is not specifically limited, examples thereof include one composed of PET or the like. While the swing lid is not specifically limited, examples thereof include one that includes a lid portion, a hinge, and a grip portion of a lid body composed of PET or the like and a rib of the lid body composed of a rubber or the like. While the sheet lid is not specifically limited, examples thereof include one that includes a lid body sheet composed of PET or the like and an adhesive seal composed of an adhesive agent containing at least one selected from the group consisting of silicone-based resins and fluorine-based resins.

While the second lid is not specifically limited insofar as the lid closes the second opening portion, for example, a lid removable from the second opening portion to open the second opening portion may be used in some embodiments, and especially, a cap attachable to and removable from the second opening portion like the second lid according to the first and the second embodiments may be used in some embodiments. This is because the second opening portion can be closed by attaching the cap and the second opening portion can be opened by removing the cap. While the material of the cap is not specifically limited, examples thereof include PET (polyethylene terephthalate).

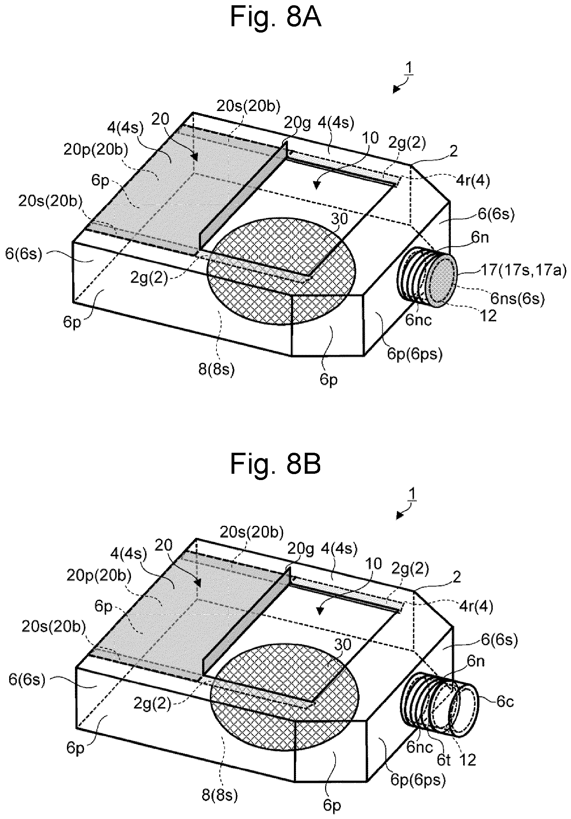

The second lid may be, for example, as illustrated in FIG. 8A, a seal lid 17 that is attached to the surface 4s of the top portion 4 or the surface 6s (for example, a surface 6ns of the neck portion 6n) of the body 6 to close the second opening portion 12, and the seal lid 17 may be removable from the second opening portion 12 by peeling off from the surface 4s of the top portion 4 or the surface 6s of the body 6. The seal lid 17 is a lid that includes a substrate sheet 17s having a size enough to cover the second opening portion 12 and an adhesive agent 17a provided on an outer peripheral portion of one surface of the substrate sheet 17s. The adhesive agent 17a is adhered to the peripheral portion around the second opening portion 12 in the surface 4s of the top portion 4 or the surface 6s (for example, the surface 6ns of the neck portion 6n) of the body 6 such that the leakage of the culture fluid can be avoided. Examples of the seal lid 17 include an aluminum seal lid in which the substrate sheet 17s is an aluminum foil. Furthermore, the second lid may be, for example, as illustrated in FIG. 8B, a lid member 6c integrally formed with the top portion 4 or the body 6 (for example, the neck portion 6n), and the lid member 6c may be removable from the second opening portion 12 by cutting off from the body 6 (for example, the neck portion 6n) along a slit 6t. Although not specifically limited, the material of the lid member 6c is usually the same as the material of the vessel main body portion 2.

The culture vessel may further include a microorganism capturing filter disposed in an area in which the microorganisms fall via the first opening portion on the bottom surface inside the vessel main body portion in some embodiments. While the microorganism capturing filter is not specifically limited insofar as the microorganism capturing filter is a porous membrane capable of capturing microorganisms, consisting of a material without a negative effect on microorganisms, and having a structure without a negative effect on microorganisms, for example, a membrane filter or the like may be used in some embodiments, and especially, a membrane filter having a filter hole size of 0.45 µm or less or the like may be used in some embodiments. This is because one having the filter hole size of 0.45 µm or less easily captures microorganisms. The microorganism capturing filter may be, for example, a circular membrane filter having a diameter of 47 mm in some embodiments. Specific examples of the membrane filter include Durapore (membrane filter made of PVDF (polyvinylidene fluoride)) manufactured by Merck, MF-Millipore (membrane filter made of MCE (mixed cellulose ester)) manufactured by Merck, Hydrophobic PTFE (membrane filter made of PTFE (polytetrafluoroethylene)) manufactured by Merck, Mitex (membrane filter made of PTFE) manufactured by Merck, LCR (membrane filter made of PTFE) manufactured by Merck, Omnipore (membrane filter made of PTFE) manufactured by Merck, Millipore Express (membrane filter made of PES (polyethersulfone)) manufactured by Merck, and Isopore (membrane filter made of PC (polycarbonate)) manufactured by Merck.

The microorganism capturing filter may be, for example, one not containing an antimicrobial substance, such as a coating with metal ions, in some embodiments, from a perspective of the material without a negative effect on microorganisms and the structure without a negative effect on microorganisms. The microorganism capturing filter may be consisting of a material that does not inhibit the culture of microorganisms in the liquid medium in some embodiments, and from this perspective, for example, a membrane filter made of mixed cellulose ester may be used in some embodiments. Furthermore, the microorganism capturing filter may be consisting of a material to which microorganisms do not easily attach in some embodiments because the culture of microorganisms can be promoted, and from this perspective, for example, a membrane filter made of PVDF and a membrane filter made of PET (polyethylene terephthalate) may be used in some embodiments.

The culture vessel may be one in which the microorganism capturing filter is attached to the area in some embodiments. This is because the microorganisms reliably fall on the microorganism capturing filter and can be captured. Especially, the culture vessel may be one in which the microorganism capturing filter is attached to the area with an adhesive agent and the adhesive agent contains at least one selected from the group consisting of silicone-based resins and fluorine-based resins in some embodiments. This is because the silicone-based resins and the fluorine-based resins are adhesive agents providing high material stability, and such adhesive agents have a low possibility of dissolving into the liquid medium and causing a negative effect on the culture.

The culture vessel may be, for example, one in which a vent hole is provided at at least one of the top portion or the body and an air-permeable membrane material that allows air to pass through without allowing a liquid to pass through is provided to cover the vent hole in some embodiments like the culture vessel according to the modifications of the first and the third embodiments. This is because the microorganisms easily fall in the inside of the vessel main body portion via the first opening portion and the microorganisms in the environment can be reliably collected. Further, this is because aerobic bacteria can be collected as the microorganisms, and the culture of the aerobic bacteria can be promoted when the microorganisms are cultured in the liquid medium. While the air-permeable membrane material is not specifically limited insofar as the air-permeable membrane material allows air to pass through without allowing a liquid to pass through, for example, a hydrophobic filter may be used in some embodiments. Here, the "hydrophobic filter" means a hydrophobic porous membrane that allows air to pass through without allowing a liquid to pass through. While the hydrophobic filter is not specifically limited insofar as the hydrophobic filter is such a membrane, examples thereof include a membrane filter made of PTFE (polytetrafluoroethylene) and a polycarbonate track-etched (PCTE) membrane filter.

The culture vessel may be one further including an air-impermeable cover that opens and closes the vent hole and does not allow air to pass through in some embodiments among those provided with the vent hole and the air-permeable membrane material as described above. This is because when anaerobic bacteria are collected as the microorganisms and the microorganisms are cultured in the liquid medium, by closing the vent hole with the air-impermeable cover, it can be avoided that the culture of the anaerobic bacteria is inhibited. While the air-impermeable cover is not specifically limited insofar as the air-impermeable cover can open and close the vent hole, for example, a one-sided adhesive sheet including a substrate sheet and an adhesive layer provided at one surface of the substrate sheet like the air-impermeable cover according to the modifications of the first and the third embodiments may be used in some embodiments. While the material of the substrate sheet of the air-impermeable cover is not specifically limited, examples thereof include a material same as the material of the vessel main body portion.

### 2. Culture Kit

The culture kit according to the embodiment is, for example, like the culture kit according to the third embodiment, a culture kit used for a falling bacteria test of microorganisms, and includes a culture vessel, a microorganism capturing filter, and an attachment material used for attaching the microorganism capturing filter to an area in which the microorganisms fall via the first opening portion on a bottom surface inside the vessel main body portion of the culture vessel. The culture vessel included in the culture kit is a culture vessel without a microorganism capturing filter among the culture vessels according to the embodiments.

While the attachment material is not specifically limited, the attachment material may be one including an adhesive agent containing at least one selected from the group consisting of silicone-based resins and fluorine-based resins as the adhesive agent used for attaching the microorganism capturing filter to the area in some embodiments. This is because the silicone-based resins and the fluorine-based resins are adhesive agents providing high material stability, and have a low possibility of causing a negative effect on the culture. The attachment material may be, especially, a double-sided adhesive sheet or the like including a substrate sheet and two adhesive layers provided on respective both surfaces of the substrate sheet, in which the two adhesive layers contain an adhesive agent, in some embodiments.

### 3. Method for Performing Falling Bacteria Test of Microorganisms

A method for performing a falling bacteria test of microorganisms according to the embodiment is a method for performing a falling bacteria test of microorganisms using the culture vessel according to the embodiment, and includes a step of locating the culture vessel at a test position with the first opening portion opened, a step of collecting microorganisms in an environment above the culture vessel falling in an inside of the vessel main body portion via the first opening portion until a certain period of time elapses after locating the culture vessel at the test position, a step of supplying a liquid medium to the inside of the vessel main body portion via the first opening portion or the second opening portion after collecting the microorganisms, a step of culturing the microorganisms in the liquid medium supplied to the inside of the vessel main body portion to obtain a culture fluid, a step of extracting the culture fluid to an outside of the culture vessel from the inside of the vessel main body portion via the second opening portion, and a step of testing the microorganisms using the culture fluid extracted to the outside.

While the method for performing the falling bacteria test of microorganisms is not specifically limited insofar as the method as described above is employed, the method may be a method for performing the falling bacteria test of microorganisms using a culture vessel further including a microorganism capturing filter disposed in an area in which the microorganisms fall via the first opening portion on a bottom surface inside the vessel main body portion in some embodiments, further, a method using a culture vessel in which the microorganism capturing filter is attached to the area may be used in some embodiments, and especially, a method using a culture vessel in which the microorganism capturing filter is attached to the area with an adhesive agent and the adhesive agent contains at least one selected from the group consisting of silicone-based resins and fluorine-based resins may be used in some embodiments. The method for performing the falling bacteria test of microorganisms may be a method for performing a falling bacteria test of microorganisms using a culture vessel provided with a vent hole at at least one of the top portion or the body and an air-permeable membrane material that allows air to pass through without allowing a liquid to pass through to cover the vent hole in some embodiments.

While the microorganisms as a subject of the falling bacteria test are not specifically limited, and are not specifically limited insofar as the microorganisms are generally known as microorganisms that is a subject of the falling bacteria test, examples thereof include fungi, such as mold, and bacteria.

In the step of collecting microorganisms, while the second opening portion of the vessel main body portion of the culture vessel may be closed when the microorganisms in the environment fall in the inside of the vessel main body portion via the first opening portion, the second opening portion of the vessel main body portion of the culture vessel may be opened when the microorganisms in the environment fall in the inside of the vessel main body portion via the first opening portion. In this case, since air flows from the inside of the vessel main body portion to the outside via the second opening portion, the microorganisms easily fall in the inside of the vessel main body portion via the first opening portion.

In the step of culturing the microorganisms in the liquid medium supplied to the inside of the vessel main body portion to obtain a culture fluid, while the microorganisms may be cultured in the liquid medium in a state where both of the first opening portion and the second opening portion are closed, the microorganisms may be cultured in the liquid medium in a state where at least one of the first opening portion or the second opening portion is opened. In this case, since air can be taken into the liquid medium from the outside of the vessel main body portion via at least one of the first opening portion or the second opening portion, the culture of aerobic bacteria can be promoted. Note that in this case, it is necessary to place the culture vessel with a measure to avoid flowing out of the liquid medium from the inside of the vessel main body portion to the outside via the opened one of the first opening portion and the second opening portion.

While the embodiments according to the culture vessel, the culture kit, and the method for performing a falling bacteria test of microorganisms of the present disclosure are described above in detail, the present disclosure is not limited to the embodiments described above, and various changes of design can be made without departing from the spirit of the present disclosure described in claims.

### DESCRIPTION OF SYMBOLS

- 1: Culture vessel
- 2: Vessel main body portion
- 4: Top portion
- 6: Body
- 6p: Plate-shaped member
- 6n: Neck portion
- 6h: Vent hole
- 6c: Lid member
- 6t: Slit
- 8: Bottom portion
- 10: First opening portion
- 12: Second opening portion
- 16: Cap
- 17: Seal lid
- 20: Slide lid
- 30: Microorganism capturing filter
- 32: Attachment material (double-sided adhesive sheet)
- 40: Swing lid
- 50: Sheet lid
- 60: Hydrophobic filter
- 100: Culture kit

## Claims

1. A culture vessel used for a falling bacteria test of microorganisms, comprising a vessel main body portion configured to internally house a culture fluid,
wherein the vessel main body portion has a top portion, a body, and a bottom portion,
wherein the top portion is provided with a first opening portion configured to receive the microorganisms falling from an environment above the culture vessel in an inside of the vessel main body portion,
wherein at least one of the top portion or the body is provided with a second opening portion configured to pour the culture fluid, and
wherein the culture vessel further includes a first lid that closes the first opening portion and a second lid that closes the second opening portion.

2. The culture vessel according to claim 1,
wherein the second lid is a cap attachable to and removable from the second opening portion.

3. The culture vessel according to claim 1, further comprising
a microorganism capturing filter disposed in an area in which the microorganisms fall via the first opening portion on a bottom surface inside the vessel main body portion.

4. The culture vessel according to claim 3,
wherein the microorganism capturing filter is attached to the area with an adhesive agent, and the adhesive agent contains at least one selected from the group consisting of silicone-based resins and fluorine-based resins.

5. The culture vessel according to claim 1,
wherein at least one of the top portion or the body is provided with a vent hole, and an air-permeable membrane material that allows air to pass through without allowing a liquid to pass through is provided to cover the vent hole.

6. The culture vessel according to claim 5, further comprising
an air-impermeable cover that opens and closes the vent hole, the air-impermeable cover not allowing air to pass through.

7. A culture kit used for a falling bacteria test of microorganisms, comprising:
the culture vessel according to claim 1;
a microorganism capturing filter; and
an attachment material used for attaching the microorganism capturing filter to an area in which the microorganisms fall via the first opening portion on a bottom surface inside the vessel main body portion of the culture vessel.

8. The culture kit according to claim 7,
wherein the attachment material contains an adhesive agent containing at least one selected from the group consisting of silicone-based resins and fluorine-based resins.

9. A method for performing a falling bacteria test of microorganisms using the culture vessel according to claim 1, the method comprising:
locating the culture vessel at a test position with the first opening portion opened;
collecting microorganisms in an environment above the culture vessel falling in an inside of the vessel main body portion via the first opening portion until a certain period of time elapses after locating the culture vessel at the test position;
supplying a liquid medium to the inside of the vessel main body portion via at least one of the first opening portion or the second opening portion after collecting the microorganisms;
culturing the microorganisms in the liquid medium supplied to the inside of the vessel main body portion to obtain a culture fluid;
extracting the culture fluid to an outside of the culture vessel from the inside of the vessel main body portion via the second opening portion; and
testing the microorganisms using the culture fluid extracted to the outside.
